# EUROPEAN PATENT SPECIFICATION

(11) **EP 2 049 132 B1**
(45) Date of publication and mention of the grant of the patent: **05.12.2012**
(21) Application number: 07801575.7
(22) Date of filing: 09.08.2007
(51) Int. Cl.: A61K 36/53, A61P 25/24

(54) **NOVEL AGENTS FOR THE TREATMENT OF DISORDERS CONNECTED TO IMPAIRED NEUROTRANSMISSION**
NEUE WIRKSTOFFE ZUR BEHANDLUNG VON ERKRANKUNGEN IN ZUSAMMENHANG MIT GESTÖRTER NEUROTRANSMISSION
NOUVEAUX AGENTS POUR LE TRAITEMENT DE TROUBLES EN RAPPORT AVEC UNE TRANSMISSION NERVEUSE ENDOMMAGÉE

(30) Priority: 09.08.2006 EP 06016659
(43) Date of publication of application: 22.04.2009
(73) Proprietor: DSM IP Assets B.V., 6411 TE Heerlen (NL)
(72) Inventor: FOWLER, Ann, 4310 Rheinfelden (CH); GORALCZYK, Regina, 79639 Grenzach-Wyhlen (DE); KILPERT, Claus, 68305 Mannheim (DE); SCHUELER, Goede, 79591 Eimeldingen (DE)
(74) Representative: Schwander, Kuno
(86) International application number: PCT/EP2007/007053
(87) International publication number: WO 2008/017484

(56) References cited:
- WO-A-01/45780
- WO-A-2006/022616
- DATABASE WPI Week 200175 Derwent Publications Ltd., London, GB; AN 2001-655977 XP002416197 & RU 2 174 005 C1 (FITO-EM AGRIC IND FIRM STOCK CO) 27 September 2001 (2001-09-27)
- DATABASE WPI Week 199521 Derwent Publications Ltd., London, GB; AN 1995-159708 XP002416198 & RU 2 019 185 C1 (RUSANOV S E) 15 September 1994 (1994-09-15)
- DATABASE WPI Week 200022 Derwent Publications Ltd., London, GB; AN 2000-254738 XP002416199 & RO 115 120 B1 (BOJOR O) 30 November 1999 (1999-11-30)
- DATABASE WPI Week 200609 Derwent Publications Ltd., London, GB; AN 2006-082996 XP002416200 & JP 2006 008555 A (DOKURITSU GYOSEI HOJIN KAGAKU GIJUTSU SH) 12 January 2006 (2006-01-12)

## Description

The present invention relates to the use of oregano extracts and/or volatile components of oregano extract as agents for use in a method of treatment of disorders connected to impaired, i.e. reduced neurotransmission as detailed herein.

It has been found, in accordance with this invention, that such extracts are serotonin re-uptake inhibitors, noradrenaline reuptake inhibitors, and dopamine reuptake inhibitors. Thus, they can mimic the action of pharmaceuticals which are dual uptake inhibitors and triple uptake inhibitors. Thus these extracts and their volatiles are particularly effective in treating mood disorders, including depression, and anxiety, and can be used to regulate biorhythms in humans. They also have numerous veterinary uses as well, and can be used to prevent or treat the behavioral or physiological consequences of stress.

WO 95/05838 discloses the use of a plant volatile oil derivable from clove, nutmeg, pepper, thyme, paprika, oregano, marjoram, basil and French tarragon or a constituent thereof (e.g. linalool, thujone, camphene, carvacrol and thymol from thyme oil) to combat deleterious changes in the peripheral nervous system (such as morphology, structure an quantity of tissue). There is no teaching of the use of these substances to affect biochemical processes in the brain.

WO 01/45780 discloses the use of light therapy, optionally in combination with aromatherapy, whereby origanum is one of the possible ingredients, to treat sleeping disorders combined with nervousness. Thus, the composition is administered via olfactory means.

### Field of the present invention

This invention relates to the use of oregano extracts and/or their volatile components for the manufacture of compositions for the treatment and prevention of disorders connected to impaired or reduced neurotransmission. Thus, in one aspect the present invention relates to dietary or pharmaceutical, or veterinary compositions comprising at least one oregano extract or one of their volatile components for use in a process for maintaining or improving attention and concentration, memory and the capacity for remembering, learning ability, language processing, problem solving and intellectual functioning,
improvement of short-term memory,
increasing mental alertness,
increasing the ability to focus and mental sharpness,
enhancing mental vigilance,
reducing mental fatigue,
supporting cognitive wellness,
maintaining balanced cognitive function,
regulation of hunger and satiety and the regulation of motor activity,
using as an antidepressant, a mood/vitality improver, a stress relieve, a reducer of anxiety, a reducer of tension, a reducer of sadness, a reducer of unhappiness/discontentedness, a reducer of irritability, a reducer of dysphoria, a reducer of obsessive-compulsive behavior, and/or a relaxant, or
using as a preventor or normalizer of circadian rhythm disruptions, with the proviso that the composition does not include the combination of oregano and *Crataegus* extracts.

It has been surprisingly found that the oregano extracts and their volatile components of this invention, act as serotonin re-uptake inhibitors, thus prolonging the time that serotonin is available for neurotransmission. This leads to a mood balancing and stress relieving effect. Furthermore, the oregano extracts and their volatile components of this invention are also effective as noradrenaline reuptake inhibitors, dopamine reuptake inhibitors, and/or dual reuptake inhibitors of serotonin, noradrenaline and/or dopamine, or triple reuptake inhibitors of noradrenaline, serotonin, and dopamine. Thus, they are particularly useful for treating depression, anxiety, or a combination thereof, mediated by serotonin or noradrenaline neurotransmission or a combination thereof. They may be also useful in the treatment

The main neurotransmitters are serotonin, dopamine, noradrenaline, acetylcholine, glutamate, gamma-amino-butyric acid. Those neurotransmitters of particular relevance to mood-related disorders are serotonin, noradrenaline, and dopamine. Increase in neurotransmission is achieved by increasing the concentration of the neurotransmitter in the synaptic cleft thus making it available for increased or prolonged neurotransmission through inhibition of re-uptake into the pre-synaptic nerve end, or by preventing neurotransmitter catabolism by inhibition of degrading enzymes such as monoamine oxidase A and B.

### DEFINITIONS

The terms "impaired neurotransmission" and "reduced neurotransmission" are used interchangeably throughout the present application. They are used in the present application in accordance with their meaning well-known to the person skilled in the art, and relate to a state of deregulation of neurotransmission, which may occur at the level of neurotransmitter biosynthesis, processing, storage, release, re-uptake and receptor binding. Impaired neurotransmission, in particular a reduction of neurotransmission, may manifest itself in animals including humans as a disturbance of behavior, emotions, mood and thinking processes, for example, in one of various types of depression.

The term "oregano extract and/or its volatile components" is meant to comprise not only complete mixtures of extractable compounds but also only volatile components of the plant taken alone or in any combination with each other. The most important volatile components of oregano extracts in accordance with the present invention are: carvacrol, thymol, thymoquinone and thymoquinol. Examples of additional volatile components of oregano extracts are: 4-tert-butylphenol; 2,3-diisopropyl-5-methylphenol; 2,4-diisopropyl-3-methylphenol; 2,4-diisoprpyl-5-methylphenol; 2,5-diisopropyl-3-methylphenol; 2,5-diisopropyl-4-methylphenol; 2,6-diisopropyl-3-methylphenol and p-ment-3-en-1-ol. The expression "oregano extracts" of the present invention do not encompass teas or hot aqueous extracts made from fresh or dried leaves or any other parts of *Oregano* species, as teas will only contain trace amounts of the volatiles.

Extracts obtained by steam distillation are, however, in the scope of the present invention. Such extracts generally contain volatile compounds that are not readily degraded. Distilled oils contain hardly any thymoquinone and other volatiles, since they degrade more rapidly during steam distillation. However, they can contain high amounts of carvacrol. SFCO2 extracts are especially preferred for their stability (up to 5 years in closed containers).

"Animals" includes humans, and encompasses mammals, fish and birds. Preferred are: humans, pets or companion animals, farm animals, and animals used in the fur industry.

"Farm animals" includes: fish, such as salmon and trout, aquaculture animals such as shrimp, pigs, horses, ruminants (cattle, sheep, goats) and poultry (such as geese, chickens, broilers, laying hens, quails, ducks, and turkeys). Preferred are poultry, cattle, sheep, goats and pigs.

"Pets" or "companion animals" include dogs, cats, birds, aquarium fish, guinea pigs, (jack) rabbits, hares and ferrets. Dogs and cats are preferred.

"Animals used in the fur industry" include minks, foxes, and hares.

"Dietary compositions" includes any type of nutritional product, such as (fortified) food/feed and beverages, and also includes clinical nutrition products, and dietary supplements.

"Fortification" means that at least an oregano extract or one or more volatile component(s) thereof was/were added during manufacture of the food/feed or beverage.

In humans, disorders connected to impaired or reduced neurotransmission, include: depression, anxiety, irritability, unhappiness/discontentedness, sadness, dysphoria, obsessive-compulsive behaviors, insomnia, and biorhythm abnormalities (disturbed circadian rhythms).

The composition which are used in this invention can thus be characterized as mood balancing agents, mood/vitality improvers, stress relievers, anxiety reducers, tension reducers, relaxants, sleep improvers, and normalizers of biorhythms.

Numerous pharmaceutical compositions which are neurotransmitter regulators have proven helpful in various mood-related disorders. As the extracts of this invention have been found to work using the same or similar biochemical pathways, then it can be concluded that they are useful for similar conditions as uptake inhibitors.

Compounds that increase neurotransmitter levels in the brain and thus enhance their transmission, can therefore exhibit antidepressant properties as well as beneficial effects on a variety of other mental disorders (Neurotransmitters, Drugs and Brain function, R.A. Web-ster (ed), John Wiley & Sons, New York, 2001, p. 187-211, 289-452, 477-498).

Medicaments for the treatment of disorders connected to impaired neurotransmission encompass antidepressants, mood improvers, stress relievers, condition improvers, anxiety reducers and obsessive-compulsive behaviour reducers. They all improve, enhance and support the physiological neurotransmission, especially in the central nervous system, and therefore alleviate mental malfunction.

Tricyclic antidepressant compounds (TCAs) such as imipramine, amitriptyline, and clomipramine, inhibit the re-uptake of serotonin, noradrenaline and/or dopamine. They are widely regarded as among the most effective antidepressants available, but they have a number of disadvantages because they additionally interact with a number of brain receptors, e.g., with cholinergic receptors. Most importantly, TCAs are risky because, taken in overdose, they frequently show acute cardiotoxicity.

Another class of antidepressant drugs are the so-called SSRIs (selective serotonin re-uptake inhibitors), including fluoxetine, paroxetine, sertraline, citalopram, and fluvoxamine, that block the serotonin transporter (SERT), a high affinity sodium chloride-dependent neurotransmitter transporter that terminates serotonergic neurotransmission by reuptake of serotonin. They have been proven to be effective in the treatment of depression and anxiety, and are usually better tolerated than TCAs. These medications are typically started at low dosages and may be increased until they reach a therapeutic level. A common side effect is nausea. Other possible side effects include decreased appetite, dry mouth, sweating, infection, constipation, yawn, tremor, sleepiness and sexual dysfunction.

Also, systematic reviews and placebo-controlled randomized clinical trials (RCTs) indicate that some SSRIs (escitalopram. Paroxetine and sertraline), the SNRI venlafaxine, some benzodiazepines (alprazolam and diazepam), the tricyclic antidepressant imipramine, and the 5-HT_{1A} partial agonist buspirone are all efficacious in acute treatment. In general, the effect of treatment is often moderate and symptoms reappear when the treatment period is discontinued. Therefore, a continuous long-term treatment or prevention with compounds which have fewer side effects than SSRIs and can be taken over long time periods might be favorable over drug treatment. This can be achieved by supplementing persons at need with oregano extracts or their volatile components in the form of dietary supplements.

Therefore, there is still a need for compounds for the method of improving of disorders connected to impaired neurotransmission which do not show the negative side effects of known antidepressants. Many patients are interested in alternative therapies with fewer or no side effects associated with the taking of known drugs. Severe depression is a long-lasting and recurring disease, which is usually poorly diagnosed. Furthermore, many patients suffer from mild or moderately severe depression. Thus, there is an increasing interest in the development of compounds, as well as of pharmaceutical and/or dietary compositions, that can be used to treat or prevent the development of mental diseases/disorders such as depression, in people at risk, and to stabilize mood.

In addition, compounds that prevent the catabolism of neurotransmitters more broadly by inhibiting the monoamineoxidases (MAOs) A and B, so-called MAO-inhibitors (MAOIs), exhibit antidepressant effects. MAOs catalyse the oxidation of amine group-containing neurotransmitters such as serotonin, noradrenaline, and dopamine.

Furthermore, modulators of neurotransmission exert pleiotropic effects on mental and cognitive functions. In addition to depression, examples of other human conditions are listed below.

### General Anxiety Disorder (GAD) and other Anxiety Disorder

It is well known that impaired neurotransmission, e.g. low neurotransmitter levels, is connected to various mental diseases and conditions such as depression and generalized anxiety disorders (GAD). Patients often suffer, either as a comorbidity to depression or alone, from generalized anxiety syndrome or generalized anxiety disorder. GAD is a highly prevalent anxiety condition and chronic illness in primary care (∼10% of patients) (Wittchen et al, 2005 Eur. Neuropsychopharm. 15: 357-376). Patients present to their primary care physician with multiple physical symptoms. GAD is characterized by chronic tension, and anxious worrying and tension (>6 months), which are disabling and uncontrollable, and accompanied by a characteristic hypervigilance syndrome (including restlessness, muscle tension, and sleep problems). If untreated, GAD runs a chronic, fluctuating course and tends to become more severe with age. GAD patients suffer from sub-syndromal depression. GAD patients are classified as high utilizers with the highest overall direct and indirect health economic burden of all anxiety and depressive disorders. Despite high GAD incidence, few sufferers are diagnosed, prescribed medication, or receive psychiatric referral; simple diagnostic tools to aid patient recognition and monitoring are needed. Regardless of specific diagnosis, physicians require effective GAD-symptom treatments. SSRIs such as paroxetine, are effective for GAD treatment [Stocchi et al., 2003 J Clin Psychiatry 63(3):250.]

A number of SSRIs have been approved by the FDA for treatment of anxiety disorders:

| | | |
|---|---|---|
| fluoxetine | - | OCD, PD |
| Sernaline | - | OCD, PD, PTSD, SAD |
| paroxetine | - | OCD, PD, SAD, GAD, PTSD |
| escitalopram | - | GAD |
| fluvoxamine | - | OCD |

| | | |
|---|---|---|
| [OCD: obsessive-compulsive disorder, PD: panic disorder, PTSD: post-traumatic stress disorder, SAD: social anxiety disorder, GAD: generalised anxiety disorder] | | |

### Aggression

Pathological impulsive aggressivity may be associated with lower serotonergic innervation in the anterior cingulate cortex, as demonstrated using PET, where [¹¹C]McN 5652 binding was shown to be significantly reduced in this brain region [Frankle et al. 2005 Am. J. Psych, 162: 915-923]. In this respect, it also must be noted that serotonin dysfunction has been correlated with impulsive and violent criminal behaviours, alcohol abuse and suicide attempts, as well as being reported in children institutionalised for aggressive behavior.

Dietary depletion of tryptophan has been demonstrated to result in an increase in aggressive responses, in a laboratory situation, whereas augmentation of tryptophan resulted in a decrease in aggressive responses. Thus, presumably, decreased serotonin is linked to an increase in aggression [Marsh et al. 2002, Neuropsychopharm 26:660-671].

### Attention deficit hyperactivity disorder (ADHD)

A number of antidepressants which affect reuptake of one or more of the monoamines are also effective in the treatment of ADHD and are a good alternative to the commonly-used stimulant medications: (I) tricyclic antidepressants (TCAs), such as imipramine, desipramine and amitriptyline, whose mechanism of action includes the blockade of noradrenaline and serotonin reuptake and downregulation of β-adrenergic receptors; (2) dual reuptake inhibitors, such as bupropion (dopamine/noradrenaline reuptake inhibitor) and venlafaxine (SNRI); (3) single reuptake inhibitors, such as atomoxetine and tomoxetine (blockade of prefrontal cortex presynaptic noradrenaline transporters) [Greydanus 2005. Ind. J. Ped., 72:953-960; Chouinard 2005 J. Psych. & Neuro., 31 (3): 168-176].

### Circadian Rhythm Disturbances

Mood disorders and occupational stress can lead to disturbances in circadian rhythms (so-called biorhythms). These conditions are often chronic and persistent. Also, deregulation of circadian rhythms induced by long-distance flights (jet-lag), .as well as by shift-work, can cause similar symptoms and distress, Therefore, use of dietary supplementation to maintain the normal circadian rhythm (that an animal or human is used to), and/or to alleviate and prevent symptoms associated with a disturbed circadian rhythm, such as impairment of cognitive function and memory; and mental and physical fatigue, is warranted to improve the overall quality of life and to benefit the vital energy of a person in need thereof.

### Sleep Disorder, Insomnia, and Chronic Fatigue Syndrome

Sleep and depression are closely linked. Sleep EEG abnormalities are usual in depression, and insomnia can lead to depression. Sleep alterations affect other biological rhythms involved in depression (Vogel et al. 1990 Neurosci Biobehav Rev 14:49-63; Mc Carley 1982. Am J Psych. 139:565-570). Antidepressants can improve sleep continuity, reduce rapid eye movement (REM) sleep and prolong slow wave (SWS) sleep (Sraner et al 2006 in Clinical Pharmacology of Sleep. S.R. Pandi-Perumal et al (eds). Birkhäuser, Basel, , pp 103-124.

TCAs are commonly used for treatment of chronic fatigue syndrome. TCAs are believed to promote stage 4, non-rapid eye movement sleep, [Craig, et al 2002 Am. Fam. Physician, 65:1083-1090].

### Obsessive-compulsive disorder (OCD)

Enhanced neurotransmission at 5-HT₂ receptors may be implicated in the therapeutic action of SSRIs in OCD; hyperactivity in the neuronal loop between the orbitofrontal cortex, head of the caudate nucleus and thalamus may be attenuated by SSRIs, due to increased activation of inhibitory 5-HT₂ receptors in the orbitofrontal cortex [Blier et al 2001 J. Psych & Neuro. 26(1):37-43]. SSRIs, such as zimeldine, fluoxetine and sertraline, have been demonstrated to be effective in the treatment of OCD [Chouinard, 2005 J. Psych & Neuro. 31(3):168-176].

### Pain

Antidepressants with differing mechanisms of action can also be effective in the treatment of pain. For example, amitriptyline and mianserin, which are potent 5-HT₂ antagonists, are used for the control of chronic pain [Blier, et al 2001. J. Psych & Neuro, 26(1): 37-43]. The noradrenaline/serotonin/dopamine reuptake inhibitor, duloxetine, is efficacious in the treatment of neuropathic pain associated with diabetic peripheral neuropathy [Chouinard, 2005. J. Psych & Neuro. 31 (3): 168-176]. This type of pain is different from that associated with inflammation. According to this invention, pain is reduced using a different mechanism than decreasing inflammation.

As an antidepressant, oregano extracts and their volatile components, as well as compositions/medicaments containing them, can be used in methods of improving mental, behavioural and emotional/affective, neurotic, neurodegenerative, eating and stress related disorders such as e.g. unipolar depression, bipolar depression, acute depression, chronic depression, subchronic depression, dysthymia, postpartum depression, premenstrual dysphoria/syndrome (PMS), climacteric depressive symptoms, aggression, attention deficit disorders (ADS), social anxiety disorders, seasonal affective disorders, anxiety/generalized anxiety disorders (GAD), fibromyalgia syndrome, chronic fatigue, sleep disorders (insomnia), post-traumatic stress disorders, panic disorders, obsessive compulsive disorders, restless leg syndrome, nervousness, migraine/primary headaches and pain in general, emesis, bulimia, anorexia nervosa, binge eating disorder, gastrointestinal disorders, bum out syndrome, irritability and tiredness.

As an antidepressant, oregano extracts and their volatile components, as well as compositions/medicaments containing them, can also be used for the manufacture of compositions for primary and secondary prevention and/or the treatment of neurocognitive impairment. Furthermore they are also effective in the treatment of depressive symptoms or other symptoms related to disturbed neurotransmission occurring as comorbidity in chronic diseases, e.g. cardiovascular diseases, strokes, cancer, Alzheimer's Disease, and Parkinson's disease. In accordance with this invention the oregano extracts and their volatile components, as well as compositions/medicaments containing them, are *not* used for treating cardiovascular diseases, strokes, cancer, Alzheimer's disease and Parkinson's disease themselves, but to treat depression evoked by these diseases.

The oregano extracts or their volatile components can be used for the manufacture of medicaments for the treatment of a disorder connected to impaired neurotransmission. They can additionally be used for the manufacture of compositions for use as mood balancing agents, mood/vitality improvers, stress relievers, condition improvers, reducers of anxiety, reducers of tension, reducers of sadness, reducers of unhappiness/discontent, reducers of irritability, reducers of dysphoria, reducers of obsessive-compulsive behaviour, relaxants, sleep improvers and/or insomnia alleviators.

"Mood improver", "vitality improver" or "emotional wellness booster" means that the mood or vitality of a person treated with it is enhanced, that his/her self esteem is increased and/or that negative thoughts and/or negative tension, sadness, unhappiness/discontent and irritability, and dysphoria are/is reduced. It also means that emotions are balanced and/or that the general, especially the mental, well-being and vitality is improved or maintained, as well as that the risk of mood swings is lessened, that the positive mood is retained, and that one feels energetic and motivated.

"Tension reducer, sadness reducer, unhappiness/discontent reducer, irritability reducer, dysphoria reducer" means that (chronic) tension and worrying are reduced or alleviated. Hypervigilance syndrome, including restlessness and muscle tension, are also reduced or relieved. Social and other phobias are also at least partially resolved. In general, the social environment is experienced as less threatening. The person is emotionally relaxed, experiences comfort and enjoys company and contact with other people. In general, the person feels energetic and motivated to conduct daily tasks.

A "relaxant" works by completely or partially correcting a person's circadian rhythm (biorhythm) which has been disturbed due to jet-lag or shift work. A relaxant will at least partially prevent or abolish the symptoms associated with such disturbances, i.e. impairment of cognitive function and memory, mental and physical fatigue, and improve overall quality of life and vital energy. Thus, the oregano extracts or one or more of their volatile component(s), may also be used to prevent and/or abolish impairment of cognitive function and memory, to prevent and/or abolish mental and physical fatigue, and to improve overall quality of life and vital energy.

A further embodiment of the present invention relates to the use of oregano extracts or their volatile components and to the use of compositions containing them as "condition improvers", i.e. as means to reduce irritability and tiredness, to reduce, prevent or alleviate physical and mental fatigue, to favour undisturbed sleep, that is to act against insomnia and sleep disorders and to improve sleep, and to increase energy in more general terms, especially to increase brain energy production, in diseased or normal healthy individuals. Moreover, such "condition improvers" are to be used for cognition improvement in general, and especially for maintenance or improvement of attention and concentration, of memory and of the capacity for remembering, of learning ability, of language processing, of problem solving and of intellectual functioning; for improvement of short-term memory; for increasing mental alertness; for increasing the ability to focus and mental sharpness, for enhancing mental vigilance; for reducing mental fatigue; for supporting cognitive wellness, for maintaining balanced cognitive function, for the regulation of hunger and satiety as well as for the regulation of motor activity.

Thus, a preferred aspect the invention relates to the use of oregano extracts and their volatile components as mood balancing agents and/or stress relievers.

In a further preferred embodiment of the present invention the oregano extracts or their volatile components, are used for maintaining circadian rhythms in humans, for alleviating and/or for preventing the symptoms associated with a disturbed circadian rhythm in humans. Thus, mood is stabilized and an emotional balance is achieved to cope with daily life stress and to maintain physical and psychological performance. Furthermore, the symptoms associated with a disturbed circadian rhythm, such as impairment of cognitive function and memory, and mental and physical fatigue, are alleviated and/or prevented so that the overall quality of life is improved. These persons also benefit from maintaining vital energy. Also, deregulation of circadian rhythms induced by long-distance flights (jet-lag) as well as by shift-work and the symptoms associated with it are alleviated and/or prevented.

Another preferred aspect of the invention relates to the use of the oregano extracts or their volatile components, for the manufacture of a composition, for use as an antidepressant.

In the context of this invention "treatment" also encompasses co-treatment as well as prevention. "Prevention" can be the prevention of the first occurrence (primary prevention) or the prevention of a reoccurrence (secondary prevention). Prevention also means that the risk of suffering from impaired neurotransmission, or from imbalanced mood or stress is reduced. The term "prevention" especially encompasses the reduction of the risk or incidence of developing certain symptoms, especially associated with a disturbed circadian rhythm.

In another embodiment, an effective dose of oregano extracts or their volatile components may especially be used for maintaining mental well-being, for maintaining a balanced cognitive function, for helping to retain a positive mood, relaxation and for supporting cognitive wellness.

The extracts or their volatile components of this invention improve, enhance and support physiological neurotransmission, especially in the central nervous system, and therefore may alleviate mental malfunction.

Yet another aspect of this invention is a method for preventing or treating disorders connected to impaired neurotransmission in humans by increasing the plasma level of carvacrol to at least 10 ng/ml in humans, preferably by increasing the plasma level of carvacrol to within the range of 10 ng/ml to 51000 ng/ml in humans. The plasma level may be measured as described in the Examples.

### VETERINARY USES

Another aspect of this invention are veterinary uses of the oregano extract and/or its volitiles. Animals may exhibit adverse behavioral and or physiological reactions to stressful situations. For example, animals raised in mass production environments, or being transported, can have a decline in meat or milk quantity or quality. Stressed poultry can resort to feather picking, reduced egg laying and cannibalism. Many animals can become aggressive or display obsessive-compulsive behaviors. The extracts and volitiles of this invention, by acting on neurotransmitters, can relieve these unwanted behaviours and physiologies in animals.

In a preferred embodiment of the present invention the oregano extracts or their volatile components, are administered for preventing stress in farm animals, in mass production livestock husbandry, during transport to slaughter and/or for preventing quality loss of meat of said farm animals during transport to slaughter.

In another preferred embodiment of the present invention the oregano extracts or their volatile components, are administered to pets or companion animals for reduction of stress, tension and aggressiveness and compulsive behavior exhibited under stressful conditions, such as separation, change or loss of the owner, during holiday separation and husbandry in so called "animal hotels", husbandry in animal shelters or refuges.

Especially pet animals and farm animals can be in conditions in need of enhanced or improved neurotransmission. Such conditions e.g. occur after capture or transport or with housing, when the animals develop analogous disorders and are distressed or aggressive, or display stereotypic behaviours, or anxiety, tension, sadness, unhappiness/discontent and irritability, dysphoria and obsessive-compulsive behaviour.

Thus, the oregano extracts or their volatile components, and the preferences as given above, can be used in general as antidepressants for animals including humans, preferably for humans and other mammals, particularly pet animals and farm animals.

Another embodiment of this invention is method for preventing stress in farm animals in mass production livestock husbandry, during transport to slaughter and/or for preventing quality loss of meat of said farm animals during transport to slaughter, comprising administering an effective dose of an oregano extract or one or more of its volatile components to farm animals which are in need thereof. The farm animals are preferably poultry, cattle, sheep, goats and swine.

In another preferred embodiment of the present invention the oregano extracts or their volatile components, are administered to poultry for preventing feather picking and cannibalism resulting e.g. in losses of meat quality and egg production. Thus, another aspect of this invention is a method for preventing loss of egg production, feather picking and cannibalism and losses of meat quality upon transport stress amongst poultry, comprising administering an effective dose of an oregano extract or one or more of its volatile components to poultry which is in need thereof.

Another aspect of this invention is a method for preventing and/or alleviating stress in aquaculture comprising the step of administering an effective dose of an oregano extract or one or more of its volatile components to animals which are in need thereof, wherein the animals are fish or shrimp.

Yet another aspect of this invention is a method for reducing stress, tension, aggressiveness and/or compulsive behavior in pet animals under stressful conditions, such as separation, change or loss of the owner, during holiday separation and husbandry in so-called "animal hotels", husbandry in animal shelter stations and other conditions of dense husbandry and breeding, comprising the step of administering an effective dose of an oregano extract or one or more of its volatile components to pet animals which are in need thereof, especially to cats and dogs which are in need thereof.

Still another aspect of this invention is a method for preventing and/or reducing symptoms associated with stressful conditions in animals used for the fur industry, preferably for minks, foxes and/or hares.

For animals, the oregano extracts or their volatile components, are in preferably administered as fortified feed or fortified beverages (e.g. as addition to the drinking water).

### Oregano extracts and their volatile components

The oregano extracts may be of any origin from a plant (whole plant or parts thereof) belonging to the genera *Origanum* such as *Origanum vulgare* and *Thymus* such as *Thymus vulgaris* in form of a concentrate of extractable compounds, especially volatile compounds. Further examples of plants from the genus *Origanum* covered by the term "oregano", are *O. majorana, O. dictamus, O. creticum, O. x majoricum, O. aureum, O. compactus, O. syriaca, O. tytthantum, O. heracleoticum, O. smyrnaeum and O. virens.* Further examples of plants from the genus *Thymus* covered by the term "oregano" are *T. herbus-barona, T. citriodorus, T. mastichiana, T. pulegioides, T. serpyllum, T. pallasianus* and *T*. *praecox.* The concentrate may still contain solvents used for the extraction, be free from them or may be transferred to specific carrier materials. The extracts may be obtained in accordance with methods well-known in the art, e.g., by (an) extraction with solvents like methanol, ethanol, ethyl acetate, diethylether, n-hexane, methylenechloride, or with super-critical fluids like carbon dioxide (pure or in mixture with other solvents such as alcohols) or dinitrogen oxide, (b) hydrodistillation for obtaining essential oils or (c) extraction/distillation with hot gases like nitrogen.

Preferably oregano extracts are used that are obtained by an extraction with the use of supercritical carbon dioxide. Such extracts have the advantage that they do not contain any organic solvents, no proteins and no heavy metals. If desired, an extraction with super-critical carbon dioxide is followed by a second supercritical fluid CO2-extraction step to remove waxes and selectively enrich the volatiles.

The oregano extracts or their volatile components can be of natural or synthetic or mixed (viz. partly natural, partly synthetic) origin, i.e., they can, apart from being obtained by extraction of plants and fractionation, be chemically synthesized and, if desired, mixed together in any desired quantities. They can be prepared and used in any desired purities and concentrations, e.g. as solutions containing them in concentrations as low as, e.g., 10% (w/w) or less, or up to nearly 100% (w/w).

Preferred are oregano extracts containing a high proportion of at least one of their volatile components. More preferred are oregano extracts containing at least a total of 70 weight-% of volatile components as mentioned above, based on the total weight of the extract. Completely natural oregano extracts may be fortified with at least one specific volatile component thereof.

Preferred oregano extracts in the context of the present invention are those wherein:
the oregano extract comprises at least 30 weight-% of carvacrol,
the oregano extract comprises at least 50 weight-% of carvacrol,
more preferably wherein the oregano extract comprises at least 60 weight-% of carvacrol, and most preferably wherein the oregano extract comprises at least 65 weight-% of carvacrol, based on the weight of the oregano extract.

Also preferred are oregano extracts are oregano extracts which comprise thymoquinone in an amount in the range of from 0 to 30 weight-%,
preferably wherein the oregano extract comprises at least weight-% of thymoquinone, more preferably wherein the oregano extract comprises at least 2 weight-% of thymoquinone,
even more preferably wherein the oregano extract comprises at least 5 weight-% of thymoquinone, and
most preferably wherein the oregano extract comprises thymoquinone in a range of from 5 to 30 weight-%, based on the weight of the oregano extract.

Other preferred oregano extracts are those wherein the oregano extract comprises at least 50 weight-% of carvacrol and from 0 to 25 weight-%, of thymoquinone,
preferably wherein the oregano extract comprises at least 50 weight-% of carvacrol and at least I weight-% of thymoquinone;
more preferably wherein the oregano extract comprises at least 55 weight-% of carvacrol and at least 2 weight-% of thymoquinone,
even more preferably wherein the oregano extract comprises at least 60 weight-% of carvacrol and at least 5 weight-% of thymoquinone, and
most preferably wherein the oregano extract comprises at least 65 weight-% of carvacrol and thymoquinone in a range of from 5 to 25 weight-%, based on the weight of the oregano extract.

Also preferred are the methods where single volatile components or their mixtures are used, whereby the volatile components are selected from the group consisting of carvacrol, thymoquinone, p-cymene, thymoquinol, limonene, linalool, borneol, 4-terpineol, thymol and .caryophyllene. Preferably the volatile components are selected from the group consisting of carvacrol, thymoquinone and p-cymene, more preferably wherein the volatile components carvacrol and/or thymoquinone, most preferably wherein the volatile component is carvacrol.

Even more preferred are oregano extracts that do not contain: a hydrophilic extract, an essential amount of one of the following constituents: rosmarinic acid, eugenol, eugenol salts, eugenol isomers, yeast cell walls or .1-piperoylpiperidine. An "essential amount" as used herein the total amount of any of these ingredients, if present at all. is preferably below 0.5 weight-%, more preferably below 0.2 weight-%, even more preferably below 0.1 weight-%, based on the total weight of said oregano extract or oregano material or the volatile component(s).

Further, certain combinations of plant extracts are not preferred in this invention, such as the combination of oregano extract and:
*Agaricus blazei,* nettle, artichoke, *Crataegus, Leonurus,* common yarrow, mistletoe, *Crataeg, , Herba viola tricolor, Scutellariae baicalensis,* turmeric, goldthread, barberry

The composition of the present invention is preferably in the form of nutritional composition, such as fortified food, fortified feed, or fortified beverages, or in form of fortified liquid food/feed for animals including humans.

The dietary and pharmaceutical compositions according to the present invention may be in any galenic form that is suitable for administering to the animal body including the human body, especially in any form that is conventional for oral administration, e.g. in solid form, such as (additives/supplements for) food or feed, food or feed premix, fortified food or feed, tablets, pills, granules, dragées, capsules, and effervescent formulations such as powders and tablets, or in liquid form such as solutions, emulsions or suspensions as e.g. beverages, pastes and oily suspensions. The pastes may be encapsulated in hard or soft shell capsules, whereby the capsules feature e.g. a matrix of (fish, swine, poultry, cow) gelatin, plant proteins or ligninsulfonate. Examples for other application forms are forms for transdermal, parenteral or injectable administration. The dietary and pharmaceutical compositions may be in the form of controlled (delayed) release formulations.. The compositions of the present invention are not administered nasally.

The dietary compositions according to the present invention may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellyfying agents, gel forming agents, antioxidants and antimicrobials.

Examples of food are cereal bars, dairy products, such as yoghurts, and bakery items, such as cakes and cookies. Examples of fortified food are cereal bars, and bakery items, such as bread, bread rolls, bagels, cakes and cookies. Examples of dietary supplements are tablets, pills, granules, dragées, capsules and effervescent formulations, in the form of non-alcoholic drinks, such as soft drinks, fruit juices, lemonades, near-water drinks, teas and milk-based drinks, in the form of liquid food, such as soups and dairy products (muesli drinks).

Beverages encompass non-alcoholic and alcoholic drinks as well as liquid preparations to be added to drinking water and liquid food. Non-alcoholic drinks are e.g. soft drinks, sport drinks, fruit juices, vegetable juices (e.g. tomato juice), lemonades, teas and milk-based drinks. Liquid foods are e.g. soups and dairy products (e.g. muesli drinks).

In addition to at least one oregano extract or one of its volatile components the pharmaceutical compositions according to the present invention may further contain conventional pharmaceutical additives and adjuvants, excipients or diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers, and the like. The carrier material can be organic or inorganic inert carrier material suitable for oral/parenteral/injectable administration.

For humans a suitable daily dosage of oregano extracts or their volatile components for the purposes of the present invention may be within the range from 0.001 mg per kg body weight to about 100 mg per kg body weight per day. More preferred is a daily dosage of about 0.01 to about 10 mg per kg body weight, and especially preferred is a daily dosage of about 0.05 to 5.0 mg per kg body weight.

In solid dosage unit preparations for humans, the oregano extract or its volatile components is/are suitably present in an amount from about 0.1 mg to about 1000 mg, preferably from about 1 mg to about 500 mg per dosage unit. For relief of symptoms associated with conditions as mentioned herein, the oregano extract or any of its volatile components is/are taken once or twice per day together with a meal for at least one week and up to 6-12 months. For prevention of occurrence of symptoms associated with conditions as mentioned herein and for the maintenance of a generally relaxed state, consumption on a regular basis is suitable.

In dietary compositions, especially in food and beverages for humans, the oregano extract or its volatile components is/are suitably present in an amount of from about 0.0001 (1 mg/kg) to about 5 weight-% (50 g/kg), preferably from about 0.001 % (10 mg/kg) to about 1 weight-%, (10 g/kg) more preferably from about 0.01 (100 mg/kg) to about 0.5 weight-% (5 g/kg), based upon the total weight of the food or beverage. For relief of symptoms associated with conditions as defined above, the food product is taken once or twice per day at least for one to three weeks or on a regular basis, i.e. at least once daily.

In food and drinks in a preferred embodiment of the invention the amount of the oregano extract or its volatile components is/are 10 to 30 mg per serving, i.e. 120 mg per kg food or drink. The food product is taken once or twice per day at least for one to three weeks or preferably on a regular basis of at least once daily.

For animals excluding humans, a suitable daily dosage of an oregano extract or its volatile components, for the purposes of the present invention may be within the range from 0.001 mg per kg body weight to about 1000 mg per kg body weight per day. More preferred is a daily dosage of about 0.1 mg to about 500 mg per kg body weight, and especially preferred is a daily dosage of about 1 mg to 100 mg per kg body weight. To prevent and reduce symptoms associated with stressful conditions, such as mass production livestock husbandry and fur industry husbandry or aquaculture, the product containing the oregano extract or its volatile component(s) is given over the animal's entire lifetime until slaughter. Especially in the case, where farm animals, such as poultry, cattle, sheep, goats and swine, especially cattle and swine, are transported to slaughter, they should be administered a daily dosage of about 3 to 800 mg/kg body weight of the extract and/or volitiles, preferably during transportation, more preferably at least 3 days before transportation and during transportation.

For pets, under stressful conditions as in animal shelter farms or pet shops, the product should be given for at least 1-3 weeks, or over the whole husbandry period. Under conditions of short-term stress, such as holiday separation, husbandry in animal "holiday hotels", visits to or stays in veterinarian clinics, the product may be given at least 3 days, and preferably 7 days, before the stressful event.

The invention is illustrated further by the following examples.

### Examples

### Example 1: Preparation of two O. vulgare extracts

In the Examples below, "-se" refers to phenol type oregano extract 1, obtained from and "-to " refers to terpineol type oregano extract 2 , both obtained from Flavex, Germany.

Dried leaves of *O. vulgare* were milled and extracted with supercritical carbon dioxide. The parameters of extraction were as follows: temperature of 45°C;
working pressure: 300 bar (-to) or 100 bar (-se); 17 kg (-to) and 15 kg (-se) of carbon dioxide per 1 kg of plant material were needed; the extracts were obtained in the separator by throttling the pressure to 60 bar at 30°C.
25 kg (-to) or 50 kg (-se) of plant material respectively yielded 1 kg of extract.

Extract 1 had the following composition (analysed by Gas Chromatography)
Total content of essential oil was 83% (the remaining parts are plant waxes)
Volatile components: terpinene 0.2%, cymene 2.6%, 4-terpineol 1.5%, thymoquinone, 23%., thymol 0.3%, carvacrol 62%, caryophyllenel.5%.

Extract 2 (RV 141-23) contained 80 - 90 % essential oil with a high content of terpineols including trans beta terpineol 35- 50 %, cis beta Terpineol 5- 10 %, 4-Terpineol 8 - 12 %, and a relative small amount of phenols including thymol 5 - 12 %, carvacrol < 1 %., based on the total content of essential oils.

### Example 2: Preparation of oregano extract 3

*Origanum vulgare* leaves were extracted with a solvent mixture of methyl tert-butyl ether and methanol with the volume ratio 9 : 1.

### Example 3: Preparation and composition of oregano extract 4 Flavex.

The oregano leaves were prepared according to Example 1, extract 1. Due to a different harvest lot, the composition was slighty different to extract 1
Total content of essential oil was 89.5% (the remaining parts are plant waxes)
Volatile components: cymene 7.8%, 4-terpineol 1.2%, thymoquinone, 12.1 %., thymol 0.22%, carvacrol 68.5%, caryophyllene 1.6%, limonene 0.1%, linalool 0.77%, borneol 2.6%..

### Example 4: Detailed composition of oregano extracts

Extracts were prepared by steam distillation or oregano leaves.

Composition was as follows:

### Method of analysis GC/MS

| | GC/MS | GC/MS | GC/MS |
|---|---|---|---|
| | **Carvacrol** | **Thymoquinone** | **Thymol** |
| | **w/w** | **w/w** | **w/w** |
| Extract 5 (Oregano Dragon Spice) | 61.6 | - | 9.0 |
| Extract 6 (Yafa herbs/Morocco) | 30.7 | - | 21.6 |
| Extract 7 (Aysun/Derial Turkey) | 64.9 | - | 0.3 |

### Example 5: Serotonin uptake inhibition by oregano extracts

HEK-293 cells stably expressing the human serotonin re-uptake transporter (hSERT) were obtained from R. Blakely, Vanderbilt University, USA. The cells were routinely grown in Dulbecco's Modified Eagle's Medium, purchased from Bioconcept, Allschwil, Switzerland containing 10% fetal calf serum, penicillin, streptomycin, L-glutamine and the antibiotic G418 and passaged by trypsinisation. 1 day prior to the assay cells were seeded in the above mentioned medium. Immediately prior to the assay the medium was replaced by Krebs-Ringer bicarbonate buffer, purchased from Sigma Chemicals Ltd., supplemented with 35 µM pargyline, 2.2 mM CaCl₂, 1 mM ascorbic acid and 5 mM N-2-hydroxyethylpiperazine-N'-2-ethanesulfonic acid ("Hepes" buffer). Serotonin uptake into the cells was determined by addition of radio-labeled (³H) serotonin (Amersham Biosciences GE Healthcare, Slough, UK) to a concentration of 20 nM, and incubation for 30 minutes at room temperature. Following removal of unincorporated label by gentle washing three times with the above buffer, incorporated serotonin was quantified by liquid scintillation counting.

Serotonin uptake via the transporter was inhibited by the oregano extract in a dose dependent manner. The measured IC₅₀ values for inhibition of serotonin uptake by three oregano extracts are shown in Table 1.

**Table 1: Measured IC₅₀ values for inhibition of serotonin uptake into transfected HEK-293 cells by oregano extracts 1, 2 and 3, and its volatile components thymol, carvacrol and thymoquinol. Data is shown as mean ± s.e.m., where the IC₅₀ is stated as µM for single compounds and as µg/ml for extracts.**

| **Substance** | **IC₅₀ [µM or µg/ml] for tritiated serotonin uptake** |
|---|---|
| Oregano Extract 1 | 3.2 µg/ml ± 1.4 |
| Oregano Extract 2 | 15.31 µg/ml ± 5.3 |
| Oregano Extract 3 | 66.3 µg/ml ± 3.0 |
| Oregano Extract 4 | 1.95 µg/ml ± 0.94 (n=3) |
| Oregano Extract 5 | 7.8 µg/ml |
| Oregano Extract 6 | 5.8 µ/ml |
| Oregano Extract 7 | 4.1 µg/ml |
| Thymol | 5.0 µM ± 0.4 |
| Carvacrol | 9.5 µM |
| Thymoquinol | 26.6 µM |

### Example 6: Monoamine Oxidase inhibition by oregano extract 1.

The organic amines p-tyramine or benzylamine were used as substrates for the Monoamine oxidase A (MAO-A) and B (MAO-B) enzymes respectively. The hydrogen peroxide (H₂O₂) produced by this reaction was quantified by reaction with vanillic acid, catalysed by horse radish peroxidase (HRP).

The reactions were carried out in polystyrene microtitre plates. The MAO enzymes (final concentration 2 U/ml) were mixed with either p-tyramine (Sigma, final concentration 0.5 mM) or benzylamine (Sigma, final concentration 0.5 mM) as appropriate and the chromogenic solution (containing vanillic acid (Fluka), 4-aminoantipyrine (Fluka) and horse radish peroxidase (Sigma), final concentrations 0.25 mM, 0.125 mM and 1 U/ml, respectively) in 0.2 M potassium phosphate buffer pH 7.6. The reactions were followed in a microtitre plate absorbance reader e.g. Spectramax M5 (Molecular Devices Corporation). Absorbance readings at 495 nm were taken every 15 seconds for 40 minutes and the initial reaction velocities calculated by linear regression using SOFTmaxPro (Molecular Devices Corporation).

The effect of oregano extract 1 on the monoamine oxidase enzymes was determined by its inclusion in the assay at a range of concentrations between 0.03 and 100 µM for 10 minutes prior to and during the incubation with substrate. To determine the effect of the compounds on the HRP catalysed portion of the reaction, the MAO enzyme was replaced by H₂O₂ (Molecular Probes, final concentration 0.2 mM). The reactions containing MAO-A and -B were both inhibited by oregano extract 1 in a dose-dependent manner, whilst the control reaction was unaffected. The measured IC₅₀ values for inhibition of monoamine oxidase activity by oregano extract I are shown in Table 2.

**Table 2: Measured IC₅₀ values for inhibition of Monoamine Oxidase -A and -B enzymes by oregano extract 1. Data is shown as mean ± s.e.m.**

| **Substance** | **IC₅ₒ [µg/ml] for Inhibition of MAO-A** | **IC₅₀ [µg/ml] for Inhibition of MAO-B** |
|---|---|---|
| Oregano Extract 1 | 2.7 µg/ml ± 0.8 s.e.m. n=2 | 13.4 µg/ml ± 3.6 s.e.m. n=2 |

### Example 7. Effect of oregano extract 1 in the Primary Observation (Irwin) Test in the mouse

The method, which detects the first toxic dose, the active dose-range and the principal effects of a test substance on behavior and physiological function, follows that described by Irwin (Irwin S. 1968 Psychopharma. 13: 222-257).

Mice were administered the test substance and were observed in simultaneous comparison with a control group given vehicle (non-blind conditions). Three treated groups were compared with the same control group at any one time. All animals within a treatment group were observed simultaneously.

Behavioral modifications, physiological and neurotoxicity symptoms, pupil diameter and rectal temperature were recorded according to a standardized observation grid derived from that of Irwin. The grid contains the following items: lethality*, convulsions*, tremor*, Straub*, sedation, excitation, abnormal gait* (rolling, tip-toe), jumps*, motor in-coordination*, writhes*, piloerection*, stereotypes* (sniffing, chewing, head movements), head twitches*, scratching*, respiration*, aggressiveness*, fear, reactivity to touch, muscle tone, loss of righting reflex, ptosis, exophthalmos, loss of grasping, akinesia, catalepsy, loss of traction, loss of corneal reflex, analgesia, defecation, salivation, lacrimation, pupil diameter (Unit = 1/45 mm) and rectal temperature.

Observations were performed 15, 30, 60, 120 and 180 minutes after administration of the test substance and also 24 hours later. The symptoms marked (*) were observed continuously from 0 to 15 minutes after administration. Five mice were studied per group. Oregano 4 extract was solubilized in 3 % (v/v) DMSO, 3 % (v/v) Tween 80 in saline (0.9 % w/v NaCl) and injected into mice intraperitoneally (i.p.).

### Results

- Oregano extract 1 at 3 mg/kg did not induce additional behavioral changes, as compared with the vehicle.
- At 10 mg/kg, it induced slight sedation in 3 mice and decreased fear from 15 to 30 minutes in all five mice.
- At 30 mg/kg, oregano extract 1 induced slight sedation in four or five mice from 15 to 60 minutes, decreased fear in three or four mice from 15 to 60 minutes and decreased muscle tone in one to five mice from 30 to 60 minutes.
- At 100 mg/kg, oregano extract 1 induced sedation which was slight-to-marked in four or five mice from 15 to 180 minutes and slight in one mouse at 24 hours. It decreased fear in four or five mice from 15 to 180 minutes, decreased reactivity to touch in four mice from 60 to 180 minutes and decreased muscle tone in three or four mice from 30 minutes to 24 hours.

Thus, oregano extract 1 showed a dose-dependent moderate sedative and relaxant effect and reduced fear.

### Example 8: Porsolt's swim test

The "Behavioural Despair Test" or "Porsolt's Forced Swim Test" is a validated animal model for depression (see Nagatsu, 2004 NeuroTox., 25:11-20, and Porsolt et al., 1977 Arch. Int. Pharmacodyn 229:327-336). It responds to enhancement of the transmission of several neurotransmitters including serotonin, dopamine and noradrenaline.

The test, which detects antidepressant activity, was carried out as described by Porsolt et al *supra.* Micc which are forced to swim in a situation from which they cannot escape rapidly become immobile. Antidepressants decrease the duration of immobility.

Mice were individually placed in cylinders (Height = 24 cm, Diameter = 13 cm) containing 10 cm water (22°C) from which they could not escape. The mice were placed in the water for 6 minutes and the duration of immobility during the last 4 minutes was measured.

15 mice were studied per each of the four groups. The test was performed blind, i.e. the person carrying out the experiment was different from the person injecting the mice and therefore did not know to which of the four groups each mouse belonged.

Oregano extract 1 was evaluated at 3 doses (10, 30 and 60 mg/kg), administered i.p. 30 minutes before the test, and compared with a control group, administered vehicle in the same manner. The thus administered oregano extract 4 was dissolved in vehicle (saline solution containing 3% (v/v) DMSO and 3% (v/v) Tween® 80). Venlafaxine (16 mg/kg, i.p.), as comparison substance, and imipramine (32 mg/kg, i.p.), as reference compound, were administered under the same experimental conditions.

Data were analyzed by comparing the treated groups with the control group using unpaired Student's t tests and are presented in Table 3.

**Table 3: Reduction of "immobility time" with increasing concentration of oregano extract 1.**

| TREATMENT (mg/kg) i.p., - 30 min | DURATION OF IMMOBILITY (s) | | |
|---|---|---|---|
| | Mean ± s.e.m. | % change from control | |
| Vehicle | 164.7±7.6 | - | - |
| Oregano extract 1 (10) | 163.7 ± 10.3 | -1 % | NS |
| Oregano extract 1 (30) | 137.1 ± 9.5 | -17 % | * |
| Oregano extract 1 (60) | 125.6 ± 6.5 | -24 % | *** |
| Venlafaxine (16) | 80.1 ± 11.1 | -51 % | *** |
| Imipramine (32) | 49.7 ± 7.9 | -70% | *** |

| | | | |
|---|---|---|---|
| 15 mice per group **Student's t-test:** NS = not significant; * = p < 0.05; *** = p < 0.001 | | | |

Thus, oregano extract 1 significantly reduced immobility time compared with the control group, by 17% and 24% in the intermediate and highest dose groups, respectively. Overall, there was a significant dose-dependent effect of oregano extract 1. Imipramine (32 mg/kg i.p.) and venlafaxine (16 mg/kg, i.p.), administered under the same experimental conditions, significantly reduced immobility behaviour, as compared with the vehicle control (-70% and -51 %, respectively, p < 0.001).

These results show that oregano extract 1 (60 mg/kg, i.p.) has a similar efficacy as the tricyclic antidepressant, imipramine, and the SNRI, venlafaxine, in its ability to significantly reduce depression-related behaviour.

### Example 9: Marble Burying Test as test for anxiety like or obsessive compulsive behaviour

"Defensive burying" behaviour was originally demonstrated by rats burying noxious objects, such as drinking spouts filled with a unpleasant-tasting liquid (Wilkie et al., J. Exp. Anal.Behavior 1979, 31:299-306) or shock prods (Pinel et al 1978, J. Comp.Physiol. Psych. 92:708-712). The marble burying test was devised as a modification of such a test. Poling et al. 1981 J. Exp. Anal. Behavior 1981, 35:31-44) exposed rats to individual cages each containing 25 marbles, daily for 10 or 21 consecutive days. The number of marbles buried, on each day of the 10 d period, or 24 h after the 21 d exposure, were counted. The authors reported that the burying of marbles was not determined by novelty, or due to any noxious stimuli.

Marble burying behaviour by mice is reported to be sensitive to a range of minor (e.g. diazepam) and major (e.g. haloperidol) tranquilisers (Broekkamp et al.,1986 Eur J Pharmacol. 126:223-229), in addition to SSRIs (e.g. fluvoxamine, fluoxetine, citalopram), tricyclic antidepressants (e.g. imipramine, desipramine) and selective noradrenaline uptake inhibitors (e.g. reboxetine), at doses which do not induce sedation. The model may reflect either anxiety-like- or obsessive-compulsive- behaviour (see De Boer et al, 2003 Eur. J. Pharma 463: 145-161).

The method applied here follows that described by Broekkamp et al. 1986, *supra.* Mice (n = 15 per treatment group) were individually placed in transparent plastic cages (33 x 21 x 18 cm) with 5 cm of sawdust on the floor and 25 marbles (diameter 1 cm) grouped in the centre of the cage. A second, up-turned, cage served as a lid. The number of marbles covered by sawdust (by at least two-thirds) was counted at the end of the 30-minute test period. Tests were performed by investigators blind to the drug treatment protocol.

Prior to testing, all test cages and marbles were "impregnated" by leaving 10 naive mice in each cage for 15 minutes.

Oregano extract 1 was evaluated at 10, 30 and 60 mg/kg, administered i.p. 30 minutes before the test, and compared with a vehicle control group. Oregano extract I was dissolved in a saline solution containing 3% (v/v) DMSO and 3% (v/v) Tween® 80 "vehicle". The control group were administered vehicle in the same manner, while fluoxetine (32 mg/kg), administered under the same experimental conditions, was used as a reference substance. Data were analysed by comparing treated groups with vehicle control using unpaired Student's t-tests.

### Results:

**Table 4: Effects of oregano extract 1 and fluoxetine in the marble burying test in mice**

| TREATMENT (mg/kg) i.p., - 30 min | NUMBER OF MARBLES COVERED BY SAWDUST | | |
|---|---|---|---|
| | Mean ± s.e.m. | % change from control | |
| Vehicle | 21.2 ± 1.6 | - | - |
| Oregano extract 1 (10) | 12.7 ± 2.7 | -40% | * |
| Oregano extract 1 (30) | 5.8 ± 2.6 | -73 % | *** |
| Oregano extract 1 (60) | 6.9 ± 2.6 | -67 % | *** |
| Venlafaxine (16) | 0.5 ± 0.3 | -98 % | *** |
| Fluoxetine (32) | 1.4 ± 1.0 | -93 % | *** |

| | | | |
|---|---|---|---|
| 15 mice per group Student's t-test: NS = not significant; * = p < 0.05; *** = p < 0.001 | | | |

Oregano extract 1 (10, 30 and 60 mg/kg), administered i.p. 30 minutes before the test, dose-dependently decreased the number of marbles covered, as compared with the vehicle control (-40%, p < 0.05, -73%, p < 0.001 and -67%, p < 0.001, respectively).

Fluoxetine (32 mg/kg i.p.) and venlafaxine (16 mg/kg, i.p.), administered under the same experimental conditions, nearly abolished marble burying, as compared with the vehicle control (-93% and -98 %, respectively, p < 0.001).

These results show that oregano extract 1 has a similar efficacy as the SSRI, fluoxetine, and SNRI, venlafaxine, in its ability to significantly reduce anxiety/obsessive-compulsive behaviour

### Example 10: Effect of oregano extract 1 in the Light Dark Box Test in the mouse

The method, which detects anxiolytic activity, follows that described by Crawley, 1981 Pharmacol. Biochem. Behav., 15: 695-699. Anxiolytics increase the time spent in the light compartment.

Animals were placed into the light compartment of a 2-compartment box with one half light and open (25 x 27 x 27 cm) and the other half dark and closed (20 x 27 x 27 cm). The time spent in each compartment, as well as the number of times the animal crosses from one side to the other, is scored during a 3-minute test. 15 mice were studied per group. The test was performed blind.

Oregano extract 1 was evaluated at 3 doses (10, 30 and 60 mg/kg), administered i.p. 30 minutes before the test, and compared with a vehicle control group. Oregano extract 1 was dissolved in a saline solution containing 3% (v/v) DMSO and 3% (v/v) Tween® 80 ("vehicle"). The control group was administered vehicle, venlafaxine (16 mg/kg i.p) was used as comparison substance and clobazam (16 mg/kg i.p) was used as reference substance, all being administered i.p., 30 minutes prior to the test.

**Table 5: Result of the Light Dark Box Test in the mouse**

| TREATMENT (mg/kg) i.p. -30 min | TIME SPENT IN LIGHT COMPARTMENT (s) | | | NUMBER OF CROSSINGS | | |
|---|---|---|---|---|---|---|
| | mean ± s.e.m. | % change from control | | mean ± s.e.m. | % change from control | |
| Vehicle | 55.3 ± 6.3 | | - | 4.7 ± 1.1 | | - |
| Oregano extract 1 (10) | 70.7 ± 7.5 | NS | +28% | 7.1 ± 0.9 | NS | +51% |
| Oregano extract 1 (30) | 56.8 ± 5.8 | NS | +3% | 3.2 ± 0.7 | NS | -32% |
| Oregano extract 1 (60) | 74.9 ± 7.2 | NS | +35% | 3.5 ± 0.6 | NS | -26% |
| Venlafaxine (16) | 55.2 ± 9.3 | NS | 0% | 4.4 ± 0.9 | NS | -6% |
| Clobazam (16) | 118.4 ± 3.4 | *** | +114% | 7.9 ± 0.9 | * | +68% |

| | | | | | | |
|---|---|---|---|---|---|---|
| 15 mice per group **Student's t-test**: NS = not significant; * = p < 0.05; *** = p < 0.001 | | | | | | |

Oregano extract I at 10 and 60 mg/kg increased the time spent in the light compartment, as compared with vehicle control (+28%, and +35%, p = 0.0506 respectively, close to significance). It tended to increase the number of crossings at 10 mg/kg (+51%, p = 0.0888) but had no clear effects at 30 and 60 mg/kg. Thus it may be assumed that oregano extract 1 has a moderate anxiolytic effect, as measured in the light-dark box test in the mouse.

### Example 11: Effect of oregano extract 4 in Porsolt's forced swim test in the mouse after subchronic, oral gavage

This test, which detects antidepressant activity, was performed in the same manner as that described in example 7, except that the route of administering the compound and the doses were different. Oregano extract 4 was evaluated at 3 doses (75, 150, and 300mg/kg), administered orally (p.o.) 24, 5, and I hour before the test, and compared with a vehicle control group. The thus administered oregano extract 1 was dissolved in tocopherol-stripped corn oil "vehicle"). The control group was administered vehicle (p.o.), while imipramine (32 mg/kg, p.o.; dissolved in water) was administered to a separate group as reference compound, 24, 5 and 1 h prior to the test.

Mice were individually placed in cylinders (Height = 24.5 cm, Diameter = 19.5 cm) containing 13.5 cm water (22°C) from which they could not escape. The mice were placed in the water for 6 minutes, automatically viewed via a video camera and monitored by a commercially available software system (VideoMot2, TSE Systems GmbH, Germany), and the duration of immobility during the last 4 minutes was measured.

10 mice were studied per each of the five groups. Data were analyzed by comparing the treated groups and the positive control group with the vehicle group using Analysis of Variance (ANOVA) and the Bonferroni post-hoc test.

**Table 6: Results of forced swim test in the mouse after subchronic administration of three concentrations of oregano extract 1.**

| TREATMENT (mg/kg) p.o.., - 24, -5, -1 h | DURATION OF IMMOBILITY (s) | | |
|---|---|---|---|
| | Mean ± s.e.m. | % change from control | |
| Vehicle | 129.35 ± 11.10 | - | - |
| Oregano extract 1(75) | 76.74 ± 46.02 | -41 % | * |
| Oregano extract 1(150) | 87.79 ± 14.08 | -32 % | * |
| Oregano extract 1 (300) | 102.06 ± 13.69 | -21 % | NS |
| Imipramine (32) | 64.59 ± 8.26 | -50 % | *** |

| | | | |
|---|---|---|---|
| 10 mice per group **Student's t-test:** NS = not significant; * = p < 0.05; *** = p < 0.001 | | | |

Thus, oregano extract 4 significantly reduced immobility time, compared with the control group, by 41 % and 32% in the low- and intermediate- dose groups, respectively, Overall, there was a significant effect of oregano extract 4. Imipramine (32 mg/kg i.p.), administered under the same experimental conditions, significantly reduced immobility behaviour, as compared with the vehicle control (-50%, p < 0.001).

These results show that oregano extract 4 (75 and 150 mg/kg, p.o.) has a similar efficacy as the tricyclic antidepressant, imipramine, in its ability to significantly reduce depression-related behaviour.

### Example 12: Effect of oregano extract 4 on hippocampal serotonin levels measured by in vivo microdialysis

Male Sprague-Dawley rats (250-320g) were housed in groups of 4-5 under conditions of controlled temperature (21 ± 2°C) and humidity (55 ± 10%) with free access to food and water (lights on 07.00-19.00). Rats were anaesthetised using chloral hydrate (400 mg/kg i.p.) and a single microdialysis probe (BASi type MD2200, 2 mm membrane, 30,000 dalton cut-off) implanted in the dorsal hippocampus using a stereotaxic frame at the following coordinates (rostro-caudal -4.5mm; medio-lateral -2.5mm ; dorso-ventral -4.5mm from bregma and dura surface according to Paxinos & Watson 6) and fixed in position with dental cement. Body temperature was maintained at 36°C using a heating pad and monitored via a digital rectal thermometer. The microdialysis probe was perfused with artificial cerebrospinal fluid (aCSF) at I µl/min and extracellular monoamine levels determined by collection of perfusate samples every 15 min and assayed using high-performance liquid chromatography (HPLC) with electrochemical detection.

The HPLC mobile phase (0.5mM EDTA, 0.1M monochloroacetic acid pH 3.1, 0.15g/L sodium octyl sulphate, 5% acetonitrile, 0.7% tetrahydrofuran) was pumped through the system at 70 µl/min. Monoamines were separated using a reverse-phase 1 x 100mm ODS 3mm microbore column with 5 µl injection loop and detected using a Epsilon electrochemical detector (BASi) with a glassy carbon electrode set at +650mV versus Ag/AgCl reference electrode. Dialysate peaks were identified by comparing peak elution times with reference standards and quantified according to measurement of peak area using linear regression analysis. The detection limits for 5HT and 5HLAA were defined as the sample amount producing a peak area twice that of the background noise per unit time and were approximately 0.1 fmol/sample in both cases.

Preliminary studies demonstrated that following implantation of the dialysis probe, the 5HT level was initially high due to release from platelets activated by blood clotting caused by the surgery but within 150min of completion of the surgery the basal 5HT level was almost constant. Injections were therefore routinely administered at 150, 210 and 270 min following the surgery and perfusate samples collected until 60 min following the final injection.

For routine assay, two rats were prepared of which one was used to test the oregano extract and the other a control (vehicle) sample. Because of the variability in the basal release of 5HT between assays and variability in the efficiency of the microdialysis probes to detect 5HT in the extracellular fluid, these data were normalised according to the 2 values obtained immediately prior to the first injection (namely samples at 135 and 150 min time-points). Data from replicate studies for each compound are therefore expressed as % Basal level, as is normal practice for microdialysis studies. To determine the effect of each dose of the extract on the total amount of 5HT released, the area-under-the-curve (AUC) was estimated using the trapezoid method over the sampling period following each dose administration and values for the test compound / extract compared to the appropriate control by 2-way ANOVA (test factors being Treatment and Dose) followed by post-hoc comparisons at individual doses using the Bonferroni t-test. All statistical analyses were carried out using Graphpad Prism.

Oregano extract 4 (10, 30 and 60 mg/kg, i.p.) was dissolved in saline containing 0.2% (w/v) hydroxypropylmethylcellulose, while the reference compound, fluoxetine (3, 10 and 30 mg/kg, i.p.) was dissolved in saline. Two control groups were additionally investigated, being administered saline containing 0.2% (w/v) hydroxypropylmethylcellulose or saline alone, respectively.

**Table 7: Effect of Oregano Extract I on cumulative 5HT in each dosing period and expressed relative to the basal 5HT level measured immediately before injection of the first dose of compound. Injections were performed at 150, 210 and 270 min after surgery. The dose of each compound is expressed as mg/kg. Data are expressed as mean ± s.e.m. (n=5-6). Statistical analysis for each dose of compound is compared against the corresponding time-period for the appropriate vehicle, either saline or saline + 0.2% hydroxypropylmethylcellulose using the Bonferroni t-test and for each compound overall by two-way ANOVA.**

| **TREATMENT (mg/kg)** | **Time of injection post-surgery (min)** | **5-HT LEVEL (% basal)** | | |
|---|---|---|---|---|
| | | **Mean ± s.e.m.** | **Statistical significance** | |
| | 150 | 84 ± 4 | | |
| Saline | 210 | 71 ± 9 | | |
| | 270 | 65 ± 7 | | |
| 0.2 % (w/v) hydroxypropyl-methyl cellulose in saline | 150 | 80 ± 7 | | |
| | 210 | 68 ± 4 | | |
| | 270 | 67 ± 5 | | |
| Oregano extract 4 (10) | 150 | 88 ± 3 | † | |
| Oregano extract 4 (30) | 210 | 82 ± 5 | † | |
| Oregano extract 4 (60) | 270 | 75 ± 5 | † | |
| Fluoxetine (3) | 150 | 85 ± 4 | ††† | |
| Fluoxetine (10) | 210 | 131 ± 9 | ††† | *** |
| Fluoxetine (30) | 270 | 160 ± 7 | ††† | *** |

| | | | | |
|---|---|---|---|---|
| 5 - 6 rats per group **Two-way ANOVA:** vs. saline control group, overall; †p p < 0.05, †††p < 0.001 **Bonferroni post-hoc test**: vs. saline control group; ***p < 0.001 | | | | |

Estimation of the cumulative 5HT release measured as AUC in the 60min following administration of each dose of treatment (Table 7) as analysed by 2-way ANOVA also indicated a significant overall effect of treatment with oregano extract 4 (F(1,30) = 5.61; p<0.05).

### Example 13: Dopamine uptake Inhibition by the oregano extract 1

The actions of several neurotransmitters, including dopamine, are regulated through their rapid uptake and clearance from synaptic junctions by plasma membrane transport proteins. The dopamine transporter in central dopaminergic neurones is responsible for the recovery of up to 90% of released neurotransmitter. The monoamine transporters are high affinity targets for a number of psychoactive agents such as cocaine, amphetamine, and antidepressants. These agents, by blocking transporters and consequently preventing neuronal uptake, elevate levels of extracellular neurotransmitter concentrations in both the central and peripheral nervous system, contributing to their behavioral and autonomic effects.

CHO-Ki/hDAT cells expressing the human dopamine transporter (hDAT) were plated before the assay. Cells (2 x 10⁵/ml) were incubated with oregano extract and/or vehicle in modified Tris-HEPES buffer pH 7.1 at 25°C for 20 minutes before addition of 50 nM [³H]Dopamine for 10 minutes. Specific signal was determined in the presence of 10 µM nomifensine. Cells were then solubilized with 1% SDS lysis buffer. Reduction of [³H]Dopamine uptake by 50 per cent or more (≥ 50%) relative to vehicle controls indicates significant inhibitory activity. Pure compounds (nomifensine thymoquinol) were screened at 10 concentrations up to 100 µM: 0.00316, 0.01, 0.0316, 0.1, 0.316, 1, 3.16, 10, 31.6 and 100 µM. The oregano extract was screened at 10 concentrations up to 100 µg/ml: 0.00316, 0.01, 0.0316, 0.1, 0.316, 1, 3.16, 10, 31.6 and 100 µg/ml. These same concentrations were concurrently applied to a separate group of untreated cells and evaluated for possible compound-induced cytotoxicity only if significant inhibition of uptake was observed.

**Table 8: Measured IC₅₀ values for inhibition of dopamine reuptake into transfected CHO-Ki cells by oregano extract 1, and its volatile component thymoquinol. Data is shown as mean ± s.e.m., where the IC₅₀ is stated as µM (or nM) for single compounds and as µg/ml for the oregano extract.**

| **Compound** | **IC₅₀** |
|---|---|
| *Nomifensine | 11 nM |
| thymoquinol | 65.6 ± 1.2 µM (n=2)* |
| Oregano extract 1 | 6.3 µg/ml |

| | |
|---|---|
| *Indicates standard reference agent used. | |

### References:

Giros, et al. 1992 Mol. Pharmacol. 42: 383-390
Pristupa, et al 1994 Mol. Pharmacol. 45:125-135

### Example 14: Noradrenaline uptake inhibition by the oregano extract

The actions of several neurotransmitters, including noradrenaline, are regulated through their rapid uptake and clearance from synaptic junctions by plasma membrane transport proteins. The noradrenaline transporter in central adrenergic neurones is responsible for the recovery of up to 90% of released neurotransmitter. The monoamine transporters are high affinity targets for a number of psychoactive agents such as cocaine, amphetamine, and antidepressants. These agents, by blocking transporters and consequently preventing neuronal uptake, elevate levels of extracellular neurotransmitter concentrations in both the central and peripheral nervous system, contributing to their behavioral and autonomic effects.

Human recombinant noradrenaline transporter stably expressed dog kidney MDCK cells were plated one day before the assay. The cells (2 x 10⁵/ml) were preincubated with the oregano extract and/or vehicle in modified Tri-HEPES buffer pH 7.1 at 25°C for 20 minutes, then 25 nM [³H]noradrenaline was added for 10 minutes incubation. Cells in the well were then rinsed twice, solubilized with 1% SDS lysis buffer and the lysate was counted to determine [³H]noradrenaline uptake. Specific signal was determined in the presence of 10 µM desipramine. Reduction of [³H]noradrenaline uptake by 50 percent or more (≥50%) relative to vehicle controls indicates significant inhibitory activity. Pure compounds (desipramine were screened at 10 concentrations up to 100 µM: 0.00316, 0.01, 0.0316, 0.1, 0.316, 1, 3.16, 10, 31.6 and 100 µM. The oregano extract 1 was screened at 10 concentrations up to 100 µg/ml: 0.00316, 0.01, 0.0316, 0.1, 0.316, 1, 3.16, 10, 31.6 and 100 µg/ml. These same concentrations were concurrently applied to a separate group of untreated cells and evaluated for possible compound-induced cytotoxicity only if significant inhibition of uptake was observed.

**Table 9: Measured IC₅₀ values for inhibition of noradrenaline reuptake into transfected MDCK cells by oregano extract 1. Data is shown as mean ± s.e.m., where the IC₅₀ is stated as nM for single compounds and as µg/ml for the oregano extract.**

| **Inhibitor** | **IC₅₀** |
|---|---|
| *Desipramine | 1.9 nM |
| Oregano extract | 13.6 µg/ml |

| | |
|---|---|
| *Indicates standard reference agent used. | |

### Reference:

Galli, et al. 1995. J. Exp. Biol. 198: 2197-2212

### Example 15: Measurement of the plasma level of carvacrol

The concentrations of "free" carvacrol (aglycone) and "total" carvacrol (aglycone + conjugated form) were determined in 64 rat plasma samples. 4 male and 4 female rats received a single dose of 800 mg of oregano extract 4 per kg body weight by oral gavage, respectively. The application solution was prepared in corn oil at a concentration of 200 mg extract per gram formulation. Plasma samples were collected at 0, 0.5, 1, 2, 3, 4, 6, 8, 24 hours and 48 hours (terminal) after the gavage application from at least 3 male and 3 female animals.

The sample analysis was performed with a liquid chromatography - tandem mass spectrometry (LC/MS/MS) system, using a column switching system for online cleaning and desalting of the samples.

After addition of internal standard (D2-thymol) and protein precipitation (in case of "free" analyte) or pre-digesting by β-glucuronidase followed by protein precipitation (in case of "total" analyte), the samples were injected on a Waters XTerra^{™} MS C18 guard-column used as purification column, then transferred onto a Waters XTerra^{™} C18 column. Detection was performed using tandem mass spectrometry in MRM mode.

Calibration and quality control samples were prepared in 5% Albumin bovine serum solution and human plasma. Day-to-day performance was controlled with the results of quality control (QC) samples within each analytical batch. The lower limit of quantification was set to 10.0 ng/mL for "free" carvacrol and 20.0 ng/mL for "total" carvacrol).

A kinetic study after oral administration of oregano extract 4 to male and female rats was performed.

The objective of this analytical study was the determination of "free" and "total" carvacrol concentrations in rat plasma samples. A total of 64 samples were analysed successfully.

The plasma samples were collected at different times after the gavage application. The administered dose was 800 mg/kg oregano extract 4 in corn oil.

The measured "free" carvacrol concentrations ranged from not detectable to 50100 ng/mL, and "total" carvacrol concentrations from not detectable to 50000 ng/mL.

**Table 10: Determination of free and total carvacrol in female rat plasma samples**

| **ID number** | **Sample Name (Sex / # Rat** / **Time)** | | | **Free Carvacrol (ng/mL)** | **Total Carvacrol (ng/mL)** |
|---|---|---|---|---|---|
| 1 | F | 1 | 0 | *ND | *ND |
| 2 | F | 1 | 0.5 | 30.9 | 2160 |
| 3 | F | 1 | 1 | 192 | 4220 |
| 4 | F | 1 | 2 | 64.3 | 829 |
| 5 | F | 1 | 3 | 128 | 3540 |
| 6 | F | 1 | 4 | 246 | 4480 |
| 7 | F | 1 | 6 | 135 | 4000 |
| 8 | F | 1 | 8 | 159 | 3810 |
| 9 | F | 1 | 24 | 127 | 5850 |
| 10 | F | 1 | Terminal (48h) | *ND | < LOQ = 20.0 ng/mL |
| 11 | F | 2 | 0 | *ND | *ND |
| 12 | F | 2 | 0.5 | 5120 | 14800 |
| 13 | F | 2 | 1 | 6050 | 18600 |
| 14 | F | 2 | 2 | 840 | 3550 |
| 15 | F | 2 | 3 | 228 | 1780 |
| 16 | F | 2 | 4 | 102 | 1160 |
| 17 | F | 2 | 6 | 91.9 | 1920 |
| 18 | F | 2 | 8 | 1630 | 6800 |
| 19 | F | 2 | 24 | 8.91 | 1530 |
| 20 | F | 2 | Terminal (48h) | *ND | < LOQ = 20.0 ng/mL |
| 21 | F | 3 | 0 | *ND | *ND |
| 22 | F | 3 | 0.5 | 4090 | 11000 |
| 23 | F | 3 | 1 | 3610 | 10000 |
| 24 | F | 3 | 2 | 1360 | 6470 |
| 25 | F | 3 | 3 | 174 | 1360 |
| 26 | F | 3 | 4 | 50.3 | 928 |
| 27 | F | 3 | 6 | 48.3 | 1140 |
| 28 | F | 3 | 8 | < LOQ = 10.0 ng/mL | 541 |
| 29 | F | 3 | 24 | < LOQ = 10.0 ng/mL | 1680 |
| 30 | F | 3 | Terminal (48h) | *ND | < LOQ = 20.0 ng/mL |
| 31 | F | 4 | 0 | *ND | < LOQ = 20.0 ng/mL |
| 32 | F | 4 | 0.5 | 50100 | 50000 |

| | | | | | |
|---|---|---|---|---|---|
| ND* Not detected LOQ* below lower limit of quantification: LOQ* (Free carvacrol) = 10.0 ng/mL LOQ* (Total carvacrol) = 20.0 ng/mL | | | | | |

**Table 11: Determination of free and total carvacrol in male rat plasma samples**

| **ID Number** | **Sample Name (Sex / # Rat / Time)** | | | **Free Carvacrol (ng/mL)** | **Total Carvacrol (ng/mL)** |
|---|---|---|---|---|---|
| 33 | M | 1 | 0 | *ND | *ND |
| 34 | M | 1 | 0.5 | 1320 | 18700 |
| 35 | M | 1 | 1 | 1820 | 25200 |
| 36 | M | 1 | 2 | 199 | 5590 |
| 37 | M | t | 3 | 51.5 | 3010 |
| 38 | M | 1 | 4 | 30.4 | 2750 |
| 39 | M | 1 | 6 | 83.4 | 6290 |
| 40 | M | 1 | 8 | 185 | 8610 |
| 41 | M | 1 | 24 | 68.1 | 8460 |
| 42 | M | 1 | Terminal (48h) | *ND | < LOQ = 20.0 ng/mL |
| 43 | M | 2 | 0 | *ND | *ND |
| 44 | M | 2 | 0.5 | 1530 | 11100 |
| 45 | M | 2 | 1 | 1790 | 13500 |
| 46 | M | 2 | 2 | 166 | 1260 |
| 47 | M | 2 | 3 | 58.4 | 665 |
| 48 | M | 2 | 4 | 40 | 1040 |
| 49 | M | 2 | 6 | 980 | 8080 |
| 50 | M | 2 | 8 | 156 | 2780 |
| 51 | M | 2 | 24 | < LOQ = 10.0 ng/mL | 1820 |
| 52 | M | 2 | Terminal (48h) | *ND | < LOQ = 20.0 ng/mL |
| 53 | M | 3 | 0 | *ND | *ND |
| 54 | M | 3 | 0.5 | 103 | 2310 |
| 55 | M | 3 | 1 | 1150 | 8550 |
| 56 | M | 3 | 2 | 584 | 6380 |
| 57 | M | 3 | 3 | 1580 | 11200 |
| 58 | M | 3 | 4 | 316 | 2900 |
| 59 | M | 3 | 6 | 100 | 2040 |
| 60 | M | 3 | 8 | 140 | 3630 |
| 61 | M | 3 | 24 | < LOQ = 10.0 ng/mL | 2750 |
| 62 | M | 3 | Terminal (48h) | *ND | < LOQ = 20.0 ng/mL |
| 63 | M | 4 | 0 | *ND | *ND |
| 64 | M | 4 | 0.5 | 55.9 | 4960 |

| | | | | | |
|---|---|---|---|---|---|
| ND* Not detected LOQ* below lower limit of quantification:LOQ* (Free carvacrol) = 10.0 ng/mL LOQ* (Total carvacrol) = 20.0 ng/mL | | | | | |

### Example 16: Preparation of a soft gelatin capsule

A soft gelatin capsule may be prepared comprising the following ingredients:

| **Ingredient** | **Amount per Capsule** |
|---|---|
| Oregano extract | 500 mg |
| Lecithin | 50 mg |
| Soy bean oil | 250 mg |

Two capsules per day for 3 months may be administered to a human adult for the treatment of mild chronic dysthymia.

### Example 17: Preparation of a soft gelatin capsule

A soft gelatin capsule may be prepared comprising the following ingredients:

| **Ingredient** | **Amount per Capsule** |
|---|---|
| Oregano extract | 200 mg |
| Evening primrose oil | 300 mg |
| Vitamin B₆ | 100 mg |

One capsule per day, preferably during the second half of the menstrual cycle, may be taken for 14 days for the treatment of premenstrual syndrome and premenstrual dysphoric disorder.

### Example 18: Preparation of an instant flavoured soft drink

| **Ingredient** | **Amount [g]** |
|---|---|
| Oregano extract | 0.9 |
| Sucrose, fine powder | 922.7 |
| Ascorbic acid, fine powder | 2.0 |
| Citric acid anhydrous powder | 55.0 |
| Lemon flavour | 8.0 |
| Trisodium citrate anhydrous powder | 6.0 |
| Tricalciumphosphate | 5.0 |
| β-Carotene 1% CWS from DNP AG, Kaiseraugst, Switzerland | 0.4. |
| **Total amount** | **1000** |

All ingredients are blended and sieved through a 500µm sieve. The resulting powder is put in an appropriate container and mixed in a tubular blender for at least 20 minutes. For preparing the drink, 125 g of the obtained mixed powder are mixed with sufficient water to produce one liter of beverage.

The ready-to-drink soft drink contains ca. 30 mg oregano extract per serving (250 ml).

As a strengthener and for general well-being 2 servings per day (240 ml) is recommended.

### Example 19: Preparation of a fortified non baked cereal bar

| **Ingredient** | **Amount [g]** |
|---|---|
| Oregano extract | 0.95 |
| Sugar | 114.55 |
| Water | 54.0 |
| Salt | 1.5 |
| Glucose syrup | 130.0 |
| Invert sugar syrup | 95.0 |
| Sorbitol Syrup | 35.0 |
| Palm kernel fat | 60.0 |
| Baking fat | 40.0 |
| Lecithin | 1.5 |
| Hardened palm-oil | 2.5 |
| Dried and cut apple | 63.0 |
| Cornflakes | 100.0 |
| Rice crispies | 120.0 |
| Wheat crispies | 90.0 |
| Roasted hazelnut | 40.0 |
| Skimmed milk powder | 45.0 |
| Apple flavour 74863-33 | 2.0 |
| Citric acid | 5.0 |
| **Total amount** | **1000** |

Oregano extract is premixed with skimmed milk powder and placed in a planetary bowl mixer. Cornflakes and rice crispies are added and is mixed gently. Then the dried and cut apples are added. In a first cooking pot sugar, water and salt are mixed in the amounts given above (solution 1). In a second cooking pot glucose-, invert sugar- and sorbitol- syrups are mixed in the amounts given above (solution 2). A mixture of baking fat, palm kernel fat, lecithin and emulsifier is the fat phase. Solution 1 is heated to 110°C. Solution 2 is heated to 113°C and then cooled in a cold water bath. Afterwards solutions 1 and 2 are combined. The fat phase is melted at 75°C in a water bath. The fat phase is added to the combined mixture of solutions 1 and 2. Apple flavour and citric acid are added to the liquid sugar-fat mix. The liquid mass is added to the dry ingredients and mixed well in the planetary bowl mixer. The mass is put on a marble plate and rolled to the desired thickness. The mass is cooled down to room temperature and cut into pieces. The non-baked cereal bar contains ca. 25 mg oregano extract per serving (30 g). For general well-being and energizing 1-2 cereal bars may be eaten per day.

### Example 20: Dry dog feed comprising oregano extract or its volatile components for relieving stress and revitalizing the dog

Commercial basal diet for dogs (e.g. Mera Dog "Brocken", MERA-Tiemahrung GmbH, Marienstraβe 80-84, D-47625 Kevelaer-Wetten, Germany) is sprayed with a solution of oregano extract in an amount sufficient to administer to a subject a daily dose of 50 mg per kg body weight, based on the weight of the oregano extract or its volatile components concentrate. The food composition is dried to contain dry matter of about 90 % by weight. For an average dog of 10 kg body weight to consume approx. 200g dry feed per day, the dog food contains approx. 2500 mg oregano extract or its volatile components/kg food. For heavier dogs, the feed mix is prepared accordingly.

### Example 21: Wet cat food comprising oregano extract or its volatile components

Commercial basal diet for cats (e.g. Happy Cat "Adult", Tierfeinnahrung, Südliche Hauptstraβe 38, D-86517 Wehringen, Germany) is mixed with a solution of oregano extract or its volatile components in an amount sufficient to administer to a subject a daily dose of 100 mg per kg body weight, based on the weight of the dried oregano extract or its volatile components concentrate. For an average cat of 5 kg of body weight to consume approx. 400 g of wet food, the cat food contains 1250 mg/kg oregano extract. The food composition is dried to contain dry matter of about 90 % by weight.

### Example 22: Dog treats containing oregano extract

Commercial dog treats (e.g. Mera Dog "Biscuit" for dogs as supplied by Mera Tiernahrung GmbH, Marienstrasse 80-84, 47625 Kevelaer-Wetten, Germany) are sprayed with a solution of oregano extract or its volatile components in an amount sufficient to administer to the treats 5 - 50 mg per g treats, based on the weight of the dried oregano extract or its volatile components concentrate. The food composition is dried to contain dry matter of about 90% by weight.

### Example 23: Cat treats containing oregano extract

Commercial cat treats (e.g. Whiskas Dentabits for cats as supplied by Whiskas, Master-foods GmbH, Eitzer Str. 215, 27283 Verden/Aller, Germany) are sprayed with a solution of oregano extract or its volatile components in an amount sufficient to administer to the treats 5 - 50 mg per g treats, based on the weight of the dried oregano extract or its volatile components concentrate. The food composition is dried to contain dry matter of about 90% by weight.

## Claims

1. Use of a dietary or orally administrable pharmaceutical composition comprising an extract obtained from plants of the genus *Origanum* by extraction with a solvent or super-critical fluids, hydrodistillation for obtaining essential oils, or extraction/distillation with hot gases in a process for:
maintaining or improving attention and concentration, memory and the capacity for remembering, learning ability, language processing, problem solving and intellectual functioning,
improvement of short-term memory,
increasing mental alertness,
increasing the ability to focus and mental sharpness,
enhancing mental vigilance,
reducing mental fatigue,
supporting cognitive wellness,
maintaining balanced cognitive function,
regulation of hunger and satiety and the regulation of motor activity,
using as an antidepressant, a mood/vitality improver, a stress reliever, a reducer of anxiety, a reducer of tension, a reducer of sadness, a reducer of unhappiness/discontentedness, a reducer of irritability, a reducer of dysphoria, a reducer of obsessive-compulsive behavior, and/or a relaxant, or
using as a preventor or normalizer of circadian rhythm disruptions,
with the proviso that the composition does not include the combination of oregano and *Crataegus* extracts.

2. Use of a composition according to Claim 1 wherein the oregano extract is obtained from *Origanum vulgare.*

3. Use of a composition according to any of Claims 1-2 wherein the composition is selected from the group consisting of: dairy products, yoghurts, fortified food, cereal bars, bakery items, cakes, cookies, dietary supplements, tablets, pills, granules, dragées, capsules, effervescent formulations, non-alcoholic drinks, soft drinks, sport drinks, fruit juices, lemonades, near-water drinks, teas, milk based drinks, liquid food, soups, and muesli drinks.

4. Use of a composition according to any of Claims 1-3 which is a veterinary use.

5. Use of a composition according to any of Claims 1-4 wherein the oregano extract comprises at least 30 weight% of carvacrol.

6. Use of a composition according to any of Claims 1-5 wherein the oregano extract comprises thymoquinone in an amount in the range of from 0-30 weight %.

7. Use of a composition according to any of Claims 1-6 wherein the oregano extract comprises at least 50 weight % of carvacrol and from 0-25 weight% of thymoquinone.

8. Use of a composition according to any of Claims 1-7 wherein the oregano extract contains a single volatile component selected from the group consisting of carvacrol and thymoquinone.

9. Use of a dietary or pharmaceutical composition comprising an extract obtained from plants of the genus *Origanum* by extraction with a solvent or super-critical fluids, hydrodistillation for obtaining essential oils, or extraction/distillation with hot gases in a process for :
using as a noradrenaline reuptake inhibitor, dopamine reuptake inhibitor, and/or dual uptake reinhibitors of serotonin, noradrenaline and/or dopamine, or
using as a triple uptake inhibitor of noradrenaline, serotonin and dopamine,
with the proviso that the composition does not include the combination of oregano and *Crataegus* extracts.

## Patentansprüche

1. Verwendung einer diätisch oder oral verabreichbaren pharmazeutischen Zusammensetzung, umfassend einen Extrakt, erhalten aus Pflanzen der Gattung *Origanum* durch Extraktion mit einem Lösungsmittel oder superkritischen Fluiden, Hydrodestillation zum Erhalten von essentiellen Ölen oder Extraktion/Destillation mit heißen Gasen bei einem Verfahren zum:
Aufrechterhalten oder Verbessern von Aufmerksamkeit und Konzentration, Gedächtnis und Erinnerungsvermögen, Lernfähigkeit, Sprachverarbeitung, Problemlösung und intellektueller Funktion,
Verbessern des Kurzzeitgedächtnisses,
Erhöhen der geistigen Wachheit,
Erhöhen der Fokussierungsfähigkeit und der Geistesschärfe,
Verstärken der geistigen Vigilanz,
Verringern der geistigen Müdigkeit,
Unterstützen des kognitiven Wohlbefindens,
Aufrechterhalten einer ausgeglichenen kognitiven Funktion,
Regulieren von Hunger und Sättigung und Regulieren der motorischen Aktivität,
Verwenden als Antidepressivum, Stimmungs-/Vitalitätsverbesserer, Stresslöser, Angstverringerungsmittel, Spannungsverringerungsmittel, Traurigkeitsverringerungsmittel, Betrübtheits-/Unzufriedenheitsverringerungsmittel, Reizbarkeitsverringerungsmittel, Dysphorieverringerungsmittel, Verringerungsmittel von obsessiv-kompulsivem Verhalten und/oder Beruhigungsmittel, oder
Verwenden als Verhinderungsmittel oder Normalisierungsmittel von Tagesrhythmusunterbrechungen,
mit der Maßgabe, dass die Zusammensetzung nicht die Kombination von Oregano- und *Crataegus-*Extrakten enthält.

2. Verwendung einer Zusammensetzung gemäß Anspruch 1, wobei der Oregano-Extrakt aus *Origanum vulgare* erhalten ist.

3. Verwendung einer Zusammensetzung gemäß einem der Ansprüche 1-2, wobei die Zusammensetzung ausgewählt ist aus der Gruppe bestehend aus: Milchprodukten, Joghurts, verstärkten Lebensmitteln, Cerealienriegeln, Backwaren, Kuchen, Keksen, Nahrungsergänzungen, Tabletten, Pillen, Granulat, Dragees, Kapseln, Brauseformulierungen, alkoholfreien Getränken, Softdrinks, Sportgetränken, Fruchtsäften, Limonaden, Near-Water-Getränken, Tees, Getränken auf Milchbasis, Flüssignahrung, Suppen und Müsligetränken.

4. Verwendung einer Zusammensetzung gemäß einem der Ansprüche 1-3, die eine Veterinärverwendung ist.

5. Verwendung einer Zusammensetzung gemäß einem der Ansprüche 1-4, wobei der Oregano-Extrakt wenigstens 30 Gew.-% an Carvacrol umfasst.

6. Verwendung einer Zusammensetzung gemäß einem der Ansprüche 1-5, wobei der Oregano-Extrakt Thymochinon in einer Menge im Bereich von 0-30 Gew.-% umfasst.

7. Verwendung einer Zusammensetzung gemäß einem der Ansprüche 1-6, wobei der Oregano-Extrakt wenigstens 50 Gew.-% an Carvacrol und 0-25 Gew.-% an Thymochinon umfasst.

8. Verwendung einer Zusammensetzung gemäß einem der Ansprüche 1-7, wobei der Oregano-Extrakt eine einzige flüchtige Komponente enthält, ausgewählt aus der Gruppe bestehend aus Carvacrol und Thymochinon.

9. Verwendung einer diätischen oder pharmazeutischen Zusammensetzung, umfassend einen Extrakt, erhalten aus Pflanzen der Gattung *Origanum* durch Extraktion mit einem Lösungsmittel oder superkritischen Fluiden, Hydrodestillation zum Erhalten von essentiellen Ölen oder Extraktion/Destillation mit heißen Gasen bei einem Verfahren zum:
Verwenden als Noradrenalin-Wiederaufnahmehemmer, Dopamin-Wiederaufnahmehemmer und/oder dualer Aufnahmehemmer von Serotonin, Noradrenalin und/oder Dopamin, oder
Verwenden als dreifacher Aufnahmehemmer von Noradrenalin, Serotonin und Dopamin,
mit der Maßgabe, dass die Zusammensetzung nicht die Kombination von Oregano- und *Crataegus-*Extrakten enthält.

## Revendications

1. Utilisation d'une composition alimentaire ou pharmaceutique administrable par voie orale comprenant un extrait obtenu à partir de plantes du genre *Origanum* par extraction avec un solvant ou des liquides supercritiques, hydrodistillation pour obtenir des huiles essentielles, ou extraction/distillation avec des gaz chauds dans un procédé pour :
maintenir ou améliorer l'attention et la concentration, la mémoire et la capacité de se souvenir, la capacité d'apprentissage, la gestion du langage, la résolution des problèmes et la faculté intellectuelle,
améliorer la mémoire à court-terme,
augmenter la vivacité d'esprit,
augmenter la capacité de se focaliser et l'acuité mentale,
améliorer la vigilance mentale,
réduire la fatigue mentale,
soutenir le bien être cognitif,
maintenir une fonction cognitive équilibrée,
réguler la faim et la satiété et réguler l'activité motrice,
utiliser comme antidépresseur, un agent améliorant l'humeur/la vitalité, un agent soulageant le stress, un agent réduisant l'anxiété, un agent réduisant la tension, un agent réduisant la tristesse, un agent réduisant le chagrin/le mécontentement, un agent réduisant l'irritabilité, un agent réduisant la dysphorie, un agent réduisant le comportement obsessif compulsif, et/ou un décontractant, ou
utiliser comme agent de prévention ou de normalisation des perturbations du rythme circadien,
à condition que la composition ne comprenne pas la combinaison d'extraits d'origan et de *Crataegus.*

2. Utilisation d'une composition selon la revendication 1, **caractérisée en ce que** l'extrait d'origan est obtenu à partir *d'Origanum vulgare.*

3. Utilisation d'une composition selon l'une ou l'autre des revendications 1 et 2, **caractérisée en ce que** la composition est choisie dans le groupe constitué par : les produits laitiers, les yaourts, les aliments fortifiés, les barres céréalières, les articles de boulangerie, les gâteaux, les biscuits, les compléments alimentaires, les comprimés, les pilules, les granulés, les dragées, les capsules, les formulations effervescentes, les boissons non alcoolisées, les boissons gazeuses, les boissons pour sportifs, les jus de fruits, les limonades, les boissons proches de l'eau, les thés, les boissons lactées, les aliments liquides, les soupes, et les boissons à base de muesli.

4. Utilisation d'une composition selon l'une quelconque des revendications 1 à 3, **caractérisée en ce qu'**il s'agit d'une utilisation vétérinaire.

5. Utilisation d'une composition selon l'une quelconque des revendications 1 à 4, **caractérisée en ce que** l'extrait d'origan comprend au moins 30 % en poids de carvacrol.

6. Utilisation d'une composition selon l'une quelconque des revendications 1 à 5, **caractérisée en ce que** l'extrait d'origan comprend la thymoquinone dans une quantité dans la gamme de 0 à 30 % en poids.

7. Utilisation d'une composition selon l'une quelconque des revendications 1 à 6, **caractérisée en ce que** l'extrait d'origan comprend au moins 50 % en poids de carvacrol et de 0 à 25 % en poids de thymoquinone.

8. Utilisation d'une composition selon l'une quelconque des revendications 1 à 7, **caractérisée en ce que** l'extrait d'origan contient un seul composant volatil choisi dans le groupe constitué de carvacrol et de thymoquinone.

9. Utilisation d'une composition alimentaire ou pharmaceutique comprenant un extrait obtenu à partir de plantes du genre *Origanum* par extraction avec un solvant ou des liquides supercritiques, hydrodistillation pour l'obtention d'huiles essentielles, ou extraction/distillation avec des gaz chauds dans un procédé pour :
utiliser comme inhibiteur de recapture de la noradrénaline, inhibiteur de la recapture de la dopamine, et/ou inhibiteur double de la capture de la sérotonine, de la noradrénaline et/ou de la dopamine, ou
utiliser comme inhibiteur triple de la capture de la noradrénaline, de la sérotonine et de la dopamine,
à condition que la composition ne comprenne pas de combinaison d'extraits d'origan et de *Crataegus.*
